(19) Europäisches Patentamt
European Patent Office
Office européen des brevets

(11) EP 1 688 127 B2

(12) # NOUVEAU FASCICULE DE BREVET EUROPEEN
Après la procédure d'opposition

(45) Date de publication et mention de la décision concernant l'opposition:
**10.08.2016 Bulletin 2016/32**

(45) Mention de la délivrance du brevet:
**22.04.2009 Bulletin 2009/17**

(21) Numéro de dépôt: **05292642.5**

(22) Date de dépôt: **12.12.2005**

(51) Int Cl.:
***A61K 8/34*** *(2006.01)* ***A61K 8/368*** *(2006.01)*
***A61Q 5/00*** *(2006.01)* ***A61Q 17/04*** *(2006.01)*

(54) **Composition de traitement des fibres kératiniques comprenant un alcool aromatique, un acide carboxylique aromatique et un filtre UV organique**

Haarbehandlungsmittel enthaltend ein Schutzmittel, einen aromatischen Alkohol, einen aromatischen Carbosäure und ein organische UV-filter

Hair treatment composition containing an aromatic alcohol, an aromatic carboxylic acid an organic UV filter

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **18.01.2005 FR 0550142**

(43) Date de publication de la demande:
**09.08.2006 Bulletin 2006/32**

(73) Titulaire: **L'Oréal**
**75008 Paris (FR)**

(72) Inventeurs:
- **Lalleman, Boris**
  **75015 Paris (FR)**
- **Giroud, Franck**
  **92110 Clichy (FR)**

(74) Mandataire: **Miszputen, Laurent**
**L'Oréal**
**Service DIPI**
**9 Rue Pierre Dreyfus**
**92110 Clichy (FR)**

(56) Documents cités:
**EP-A1- 1 118 319** **EP-A2- 0 521 651**
**WO-A-01/70189** **DE-A1- 4 026 756**
**DE-A1- 4 411 856** **DE-A1- 4 411 856**
**DE-A1- 10 051 773** **DE-A1- 10 219 433**
**FR-A1- 2 795 319** **GB-A- 1 096 943**
**US-A- 5 989 529** **US-A1-2002/0 182 238**

- **UMBACH W.: 'Kosmetik- Entwicklung Herstellung und Anwendung kosmetischer Mittel', vol. 2. AUFL, 1995, GEORGE THIEME VERLAG, STUTTGART page 111**
- **Material Safety Data Sheet Octyl Salicylate MSDS**



Printed by Jouve, 75001 PARIS (FR)

## Description

L'invention concerne une composition de traitement des fibres kératiniques, en particulier des cheveux, comprenant, dans un milieu aqueux physiologiquement acceptable et en particulier cosmétiquement acceptable, au moins un alcool aromatique, au moins un acide aromatique et au moins un agent protecteur particuliers ainsi que son utilisation pour le traitement desdites fibres en particulier en post-traitement d'une coloration directe ou d'oxydation.

Il est bien connu que les cheveux sont sensibilisés ou fragilisés à des degrés divers par l'action des agents atmosphériques et notamment de la lumière. De nombreuses publications divulguent que la lumière naturelle détruit certains aminoacides des cheveux. Ces agressions altérant la fibre capillaire, elles en diminuent les propriétés mécaniques comme la résistance à la traction, la charge à la rupture et l'élasticité, ou leur résistance au gonflement dans un milieu aqueux. Les cheveux sont alors ternes, rêches et cassants.

On sait également que notamment la lumière a tendance à agresser la couleur naturelle des cheveux ainsi que la couleur artificielle des cheveux teints. La couleur des cheveux s'affaiblit peu à peu ou vire vers des nuances peu esthétiques ou indésirables.

L'effet de la lumière est encore plus visible sur les cheveux teints par une coloration artificielle en particulier la coloration d'oxydation ou la coloration directe. Dans ce cas, une exposition à la lumière conduit à une dégradation des colorants présents à la fois dans le cheveu et à sa surface. Il en résulte un affadissement et/ou un virage importants de la couleur des cheveux

On recherche depuis de nombreuses années, dans l'industrie cosmétique des substances permettant de protéger les cheveux des dégradations engendrées par les agressions atmosphériques, telles que la lumière. En particulier, on recherche des produits protégeant l'intégrité des fibres kératiniques c'est-à-dire leur composition, leur état de surface, leur couleur naturelle ou artificielle, leurs propriétés mécaniques intrinsèques (la résistance à la traction, la charge à la rupture et l'élasticité, ou leur résistance au gonflement dans un milieu aqueux).

Pour lutter contre ces dégradations de la kératine des cheveux, on a déjà proposé d'utiliser des agents de protection comme les filtres UV organiques, des anti-oxydants, des chélatants ou des agents anti-radicaux libres.

On a ainsi proposé certaines substances susceptibles de filtrer les radiations lumineuses, comme l'acide 2-hydroxy-4-méthoxy benzophénone-5-sulfonique ou ses sels (FR-A-2 627 085) ou l'acide 4-(2-oxo-3-bornylidène méthyl) benzène sulfonique ou ses sels (EP-A-329 032) ou encore la lactoferrine (FR-A-2 673 839).

On connaît dans la demande de brevet japonais JP05-043437 des compositions colorantes contenant l'acide 2-hydroxy-4-méthoxy benzophénone-5-sulfonique ou ses sels, un alcool aromatique et un colorant direct acide.

Cependant, les compositions cosmétiques actuelles contenant des agents protecteurs, ne donnent pas complètement satisfaction et en particulier sur les cheveux colorés par des teintures d'oxydation bleues comme celles obtenues avec des couplages comprenant des méta-phénylènediamines.

La demanderesse a maintenant découvert de manière surprenante que des compositions aqueuses comprenant l'association d'un alcool aromatique, d'un acide carboxylique aromatique et d'un agent protecteur permettaient d'obtenir une meilleure protection des cheveux contre les effets néfastes de la lumière.

On observe notamment une amélioration de la résistance à la lumière de la coloration des cheveux teints par coloration directe ou coloration par oxydation.

Toutes ces découvertes sont à la base de la présente invention.

Ainsi, selon la présente invention, il est maintenant proposé de nouvelles compositions cosmétiques comprenant, dans un milieu aqueux physiologiquement acceptable :

a) au moins un agent protecteur des fibres kératiniques choisi parmi les filtres UV organiques dans une quantité allant de 0,2 à 20% en poids par rapport au poids total de la composition ;
b) au moins un alcool aromatique qui est un composé liquide à température ambiante et pression atmosphérique comprenant au moins un cycle benzénique ou naphtalénique et au moins une fonction alcool (OH) sur au moins un substituant dudit cycle, dans une quantité supérieure à 1% en poids par rapport au poids total de la composition ; et
c) au moins un acide carboxylique aromatique choisi parmi l'acide benzoïque, l'acide salicylique ainsi que leurs formes salifiées et leurs mélanges.

Un objet de l'invention consiste en l'utilisation d'une composition telle que définie précédemment dans la préparation d'une formulation cosmétique destinée à la protection des fibres kératiniques contre l'action des agents atmosphériques.

Un objet de l'invention consiste en l'utilisation en post traitement d'une coloration d'oxydation ou d'une coloration directe des fibres kératiniques et plus particulièrement des cheveux d'une composition telle que définie précédemment

Un autre objet de l'invention concerne un procédé de coloration consistant à appliquer sur les fibres kératiniques humaines et plus particulièrement des cheveux, une composition (A) colorante directe ou d'oxydation pendant un temps suffisant pour développer la couleur, et de faire suivre cette application, après rinçage ou non rinçage, séchage ou non

séchage, par l'application d'une composition (B) constituée d'une composition telle que définie précédemment.

Les différents objets de l'invention vont maintenant être détaillés. L'ensemble des significations et définitions des composés utilisés dans la présente invention données ci-dessous sont valables pour l'ensemble des objets de l'invention.

Le milieu physiologiquement et en particulier cosmétiquement acceptable est constitué par au moins de l'eau, au moins un alcool aromatique et au moins un acide carboxylique aromatique. Il peut contenir un ou plusieurs solvants organiques cosmétiquement acceptables différents des alcools aromatiques et des acides carboxyliques aromatiques. On peut citer notamment les alcools inférieurs en $C_1$-$C_4$, comme l'éthanol, l'isopropanol, le tertiobutanol, le n-butanol ; les polyols comme le propylèneglycol, la glycérine, le diéthylèneglycol, les éthers de polyols. On préférera plus particulièrement l'éthanol.

Les filtres UV organiques (systèmes filtrant les radiations UV) sont notamment choisis parmi les filtres hydrosolubles ou liposolubles, siliconés ou non siliconés.

Les filtres organiques sont notamment choisis parmi les dérivés de dibenzoylméthane ; les anthranilates ; les dérivés cinnamiques ; les dérivés salicyliques, les dérivés du camphre; les dérivés de la benzophénone ; les dérivés de β,β-diphénylacrylate ; les dérivés de triazine ; les dérivés de benzotriazole ; les dérivés de benzalmalonate ; les dérivés de benzimidazole ; ies imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCI :

Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP, Glyceryl PABA,
PEG-25 PABA vendu sous le nom « UVINUL P25 » par BASF,

Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane vendu notamment sous le nom commercial « EUSOLEX 8020 » par MERCK,

Dérivés salicyliques :

Homosalate vendu sous le nom « Eusolex HMS » par Rona/EM Industries,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par Haarmann et REIMER,

Dérivés de β,β-diphénylacrylate:

Octocrylene vendu notamment sous le nom commercial « UVINUL N539 » par BASF,
Etocrylene, vendu notamment sous le nom commercial « UVINUL N35 » par BASF,

Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

Dérivés du benzylidène camphre :

3-Benzylidene camphorfabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
la 2,4,6-tris-(4'-amino benzalmalonate de diisobutyle)-s- triazine.

Dérivés anthraniliques :

Menthyl anthranilate vendu sous le nom commercial commercial « NEO HELIOPAN MA » par Haarmann et REIMER,

Dérivés d'imidazolines :

Ethylhexyl Dimethoxybenzylidene Dioxoimidazoline Propionate,

Dérivés du benzalmalonate :

Polyorganosiloxane à fonctions benzalmalonate comme le Polysilicone-15 vendu sous la dénomination commerciale « PARSOL SLX » par HOFFMANN LA ROCHE

Dérivés de 4,4-diarylbutadiène :

-1,1-dicarboxy (2,2'-diméthyl-propyl)-4,4-diphénylbutadiène

Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Comme filtres UV organiques liposolubles (ou lipophiles) convenant à une mise en oeuvre dans la présente invention, on peut citer plus particulièrement :

Ethylhexyl Methoxycinnamate
Butyl Methoxydibenzoylmethane
Homosalate
Ethylhexyl Salicylate,
Octocrylene,
Benzophenone-3,
2-(4-diethylamino-2-hydroxybenzoyl)-benzoate de n-hexyle.
4-Methylbenzylidene camphor,
Ethylhexyl triazone,
Bis-Ethylhexyloxyphenol Methoxyphenyl Triazine
Diethylhexyl Butamido Triazone,
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine

Comme filtres UV organiques hydrosolubles (ou hydrophiles) convenant à une mise en oeuvre dans la présente invention, on peut citer plus particulièrement :

PABA,
PEG-25 PABA
Benzylidene Camphor Sulfonic Acid,
Camphor Benzalkonium Methosulfate
Terephthalylidene Dicamphor Sulfonic Acid
Phenylbenzimidazole Sulfonic Acid
Disodium Phenyl Dibenzimidazole Tetra-sulfonate
Benzophenone-4
Benzophenone-5

Selon l'invention, le ou les agents protecteurs des fibres kératiniques représentent de 0,2 à 20% en poids par rapport au poids total de la composition.
Selon une forme préférée de l'invention, on utilisera des agents protecteurs ayant un logP (coefficient de partage octanol/eau) inférieur à 9 et plus préférentiellement inférieur à 4.
Selon une forme particulièrement préférée de l'invention, on utilisera des agents protecteurs solubles dans le milieu aqueux de la composition, en particulier des agents protecteurs solubles à au moins 0,5% dans l'eau ou les alcools inférieurs en $C_1$-$C_4$ comme l'éthanol à 25°C. Et plus particulièrement, on utilisera des filtres UV organiques hydrosolubles comme la Benzophenone-4.
Les compositions capillaires selon l'invention comprennent au moins un alcool aromatique.
Par l'expression "alcool aromatique", on entend tout composé liquide à température ambiante et pression atmosphérique comprenant au moins un cycle benzènique ou naphtalénique et au moins une fonction alcool (OH) directement liée au cycle ou liée sur au moins un substituant dudit cycle. La fonction alcool est sur un substituant du cycle benzènique ou naphtalénique.
Parmi les alcools aromatiques utilisables dans la composition selon l'invention, on peut citer en particulier

- l'alcool benzylique
- le benzoyl isopropanol
- le benzylglycol
- le phénoxyéthanol
- l'alcool di-chloro-benzylique
- le méthylphenylbutanol
- le phénoxyisopropanol
- le phénylisohexanol
- le phénylpropanol
- l'alcool phényléthylique
- leurs mélanges.

On choisira plus particulièrement l'alcool benzylique.

Selon l'invention, le ou les alcools aromatiques seront utilisés à des concentrations supérieures à 1% en poids.

Les compositions capillaires selon l'invention comprennent également au moins un acide carboxylique aromatique choisi parmi l'acide benzoïque et l'acide salicylique ainsi que leurs formes salifiées et leurs mélanges.

Les sels des acides carboxyliques aromatiques peuvent être choisis notamment parmi les sels de métal alcalin (sodium, potassium), de métal alcalino-terreux (calcium, magnésium) ou les sels d'amines organiques ou d'ammonium.

On choisira plus particulièrement l'acide benzoïque.

Selon l'invention, le ou les acides aromatiques ou leurs sels peuvent représenter de 0,001 % à 30 % en poids, de préférence de 0,01 % à 20% en poids et plus particulièrement de 0,1 à 10% en poids par rapport au poids total de la composition.

Les compositions selon l'invention peuvent contenir en plus un ou plusieurs agents de conditionnement.

Dans le cadre de la présente invention, on entend par « agent conditionneur » tout agent ayant pour fonction l'amélioration des propriétés cosmétiques des cheveux, en particulier la douceur, le démêlage, le toucher, le lissage, l'électricité statique.

Les agents de conditionnement peuvent se présenter sous forme liquide, semi-solide ou solide tels que par exemple des huiles, des cires ou des gommes.

Selon l'invention, les agents conditionneurs peuvent être choisis parmi les huiles de synthèses telles que les poly-oléfines, les huiles végétales, les huiles fluorées ou perfluorées, les cires naturelles ou synthétiques, les silicones, les polymères cationiques non polysaccharides, les composés de type céramide, les tensioactifs cationiques, les amines grasses, les acides gras et leurs dérivés ainsi que les mélanges de ces différents composés.

Les huiles de synthèse sont notamment les polyoléfines en particulier les poly-$\alpha$-oléfines et plus particulièrement :

- de type polybutène, hydrogéné ou non, et de préférence polyisobutène, hydrogéné ou non.
  On utilise de préférence les oligomères d'isobutylène de poids moléculaire inférieur à 1000 et leurs mélange avec des polyisobutylènes de poids moléculaire supérieur à 1000 et de préférence compris entre 1000 et 15000. Atitre d'exemples de poly-$\alpha$-oléfines utilisables dans le cadre de la présente invention, on peut plus particulièrement mentionner les polyisobutènes vendus sous le nom de PERMETHYL 99 A, 101 A , 102 A, 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
  De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol,
ou les huiles végétales ou animales de formule $R_9COOR_{10}$ dans laquelle $R_9$ représente le reste d'un acide gras supérieur comportant de 7 à 29 atomes de carbone et $R_{10}$ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;

On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre

1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les agents conditionneurs préférés selon l'invention sont les polymères cationiques et les silicones.

Les polymères cationiques non saccharidiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519863.

Par polymères non saccharides, on entend les polymères ne contenant pas de liaison glycoside entre des monosaccharides.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

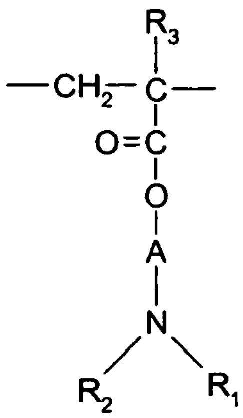 (III),

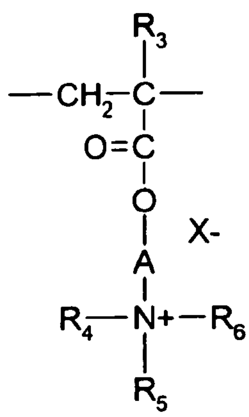 (IV)

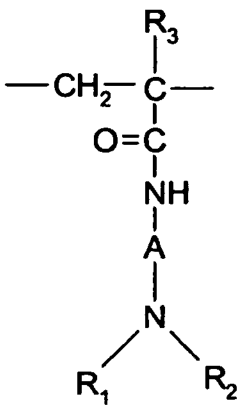
(V)

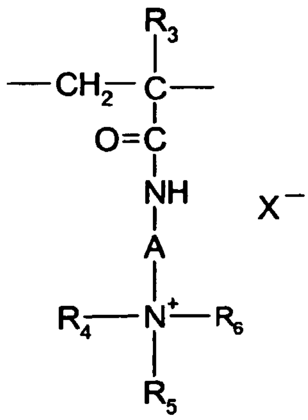
(VI)

dans lesquelles:

$R_1$ et $R_2$, identiques ou différents, représentent hydrogène ou un groupe alkyle ayant de 1 à 6 atomes de carbone et de préférence méthyle ou éthyle;
$R_3$, identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec

une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8: 1. De tels polymères sont notamment décrits dans les brevets américains 3.227.615 et 2.961.347.

Des polymères de ce type sont en particulier commercialisés sous la dénomination "Hercosett 57° par la société Hercules Inc. ou bien sous la dénomination de "PD 170" ou "Delsette 101" par la société Hercules dans le cas du copolymère d'acide adipique/époxypropyl/diéthylène-triamine.

(6) les cyclopolymères d'alkyl diallyl amine ou de dialkyl diallyl ammonium tels que les homopolymères ou copolymères comportant comme constituant principal de la chaîne des motifs répondant aux formules (VII) ou (VIII) :

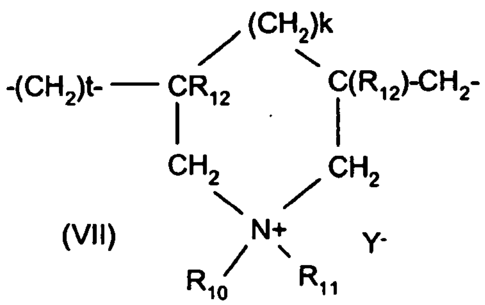
(VII)

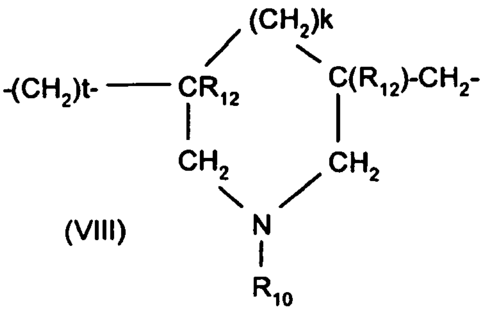
(VIII)

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. $R_{10}$ et $R_{11}$, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".

(7) le polymère de diammonium quaternaire contenant des motifs récurrents répondant à la formule :

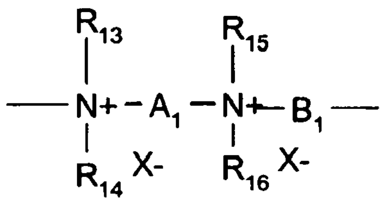 (IX)

formule (IX) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou $-CO-O-R_{17}-D$ ou $-CO-NH-R_{17}-D$ où $R_{17}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, $R_{13}$ et $R_{15}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement $(CH_2)_n-CO-D-OC-(CH_2)n-$
n est un nombre entier variant de 2 à 20 environ

dans lequel D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)_x -CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- ;

De préférence, X- est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule :

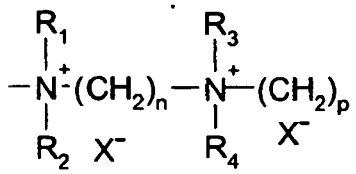 (a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique. Un composé de formule (a) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(8) les polymères de polyammonium quaternaires constitués de motifs de formule (X):

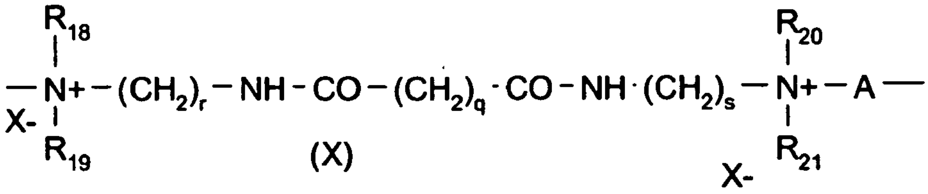

(X)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier compris entre 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers compris entre 1 et 6,
q est égal à 0 ou à un nombre entier compris entre 1 et 34,
X- désigne un anion tel qu'un halogènure,
A désigne un radical d'un dihalogénure ou représente de préférence $-CH_2-CH_2-O-CH_2-CH_2-$.

De tels composés sont notamment décrits dans la demande de brevet EP-A-122 324. On peut par exemple citer parmi ceux-ci, les produits "Mirapol®A 15", "Mirapol®AD1 ", "Mirapol®AZ1" et "Mirapol® 175" vendus par la société Miranol.

(9) Les polymères quaternaires de vinylpyrrolidone et de vinylimidazole tels que par exemple les produits commercialisés sous les dénominations Luviquat® FC 905, FC 550 et FC 370 par la société B.A.S.F.

(10) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par ia Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT

S » par la société NALCO, les polymères quaternaires de vinylpyrrolidone et de vinylimidazole et leurs mélanges.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 720" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.

On peut également citer les cyclocopolymères du type diméthylsiloxanes/méthylakylsiloxane, tel que LA "SILICONE VOLATILE FZ 3109" commercialisée par la société UNION CARBIDE, de structure chimique :

avec D :

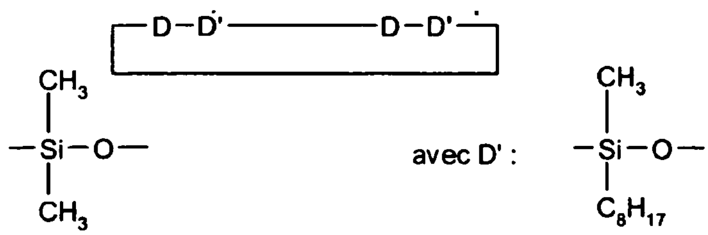

avec D' :

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à $5.10^{-6}$ $m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est par exemple mesurée à 25°C selon la norme ASTM 445 Appendice C.

Parmi ces polyalkylsiloxanes, on peut citer à titre non limitatif les produits commerciaux suivants :

- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2}m^2/s$ à 25°C.

Parmi ces polyalkylarylsiloxanes on peut citer à titre d'exemple les produits commercialisés sous les dénominations suivantes :

- •. les huiles SILBIONE de la série 70 641 de RHODIA CHIMIE ;
- •. les huiles des séries RHODORSIL 70 633 et 763 de RHODIA CHIMIE ;
- •. l'huile DOW CORNING 556 COSMETIC GRAD FLUID de DOW CORNING ;
- •. les silicones de la série PK de BAYER comme le produit PK20 ;
- •. les silicones des séries PN, PH de BAYER comme les produits PN1000 et PH1000 ;
- •. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}$ $m^2/s$.

Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radicai hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200 ;
- des groupements aminés substitués ou non comme les produits commercialisés sous la dénomination GP 4 Silicone

Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;

- des groupements thiols comme les produits commercialisées sous les dénominations "GP 72 A" et "GP 71" de GENESEE;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (XI) :

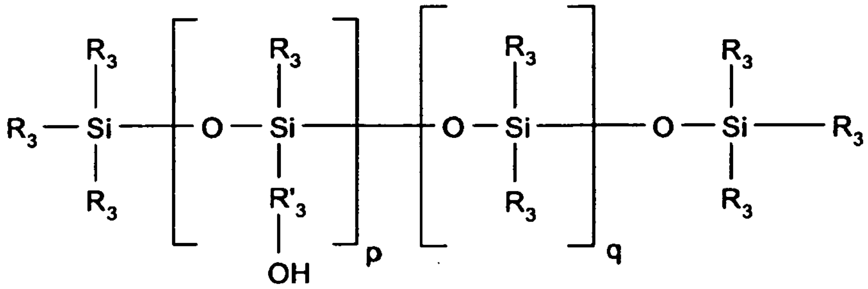

(XI)

dans laquelle les radicaux $R_3$ identiques ou différents sont choisis parmi les radicaux méthyle et phényle ; au moins 60 % en mole des radicaux $R_3$ désignant méthyle ; le radical $R'_3$ est un chaînon alkylène divalent hydrocarboné en $C_2$-$C_{18}$ p est compris entre 1 et 30 inclus ; q est compris entre 1 et 150 inclus ;

- des groupements acyloxyalkyle tels que par exemple les polyorganosiloxanes décrits dans le brevet US-A-4957732 et répondant à la formule (XII) :

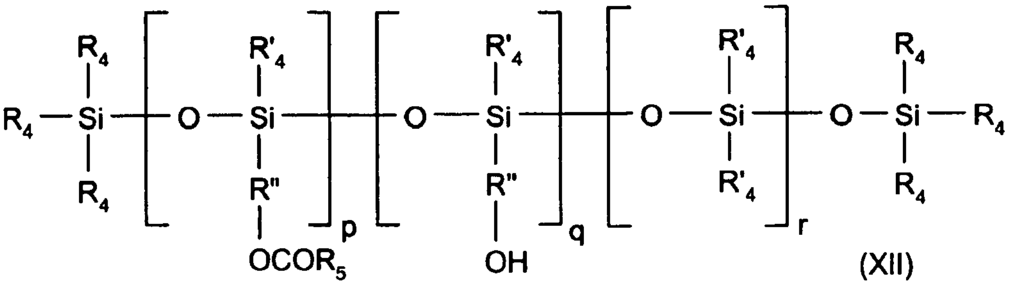

(XII)

dans laquelle :

$R_4$ désigne un groupement méthyle, phényle, -$OCOR_5$, hydroxyle, un seul des radicaux $R_4$ par atome de silicium pouvant être OH ;
$R'_4$ désigne méthyle, phényle ; au moins 60 % en proportion molaire de l'ensemble des radicaux $R_4$ et $R'_4$ désignant méthyle ;
$R_5$ désigne alkyle ou alcényle en $C_8$-$C_{20}$
R" désigne un radical alkylène hydrocarboné divalent, linéaire ou ramifié en $C_2$-$C_{18}$ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (XII) peuvent contenir des groupements :

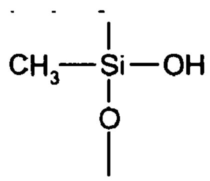

dans des proportions ne dépassant pas 15 % de la somme p + q + r.

- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

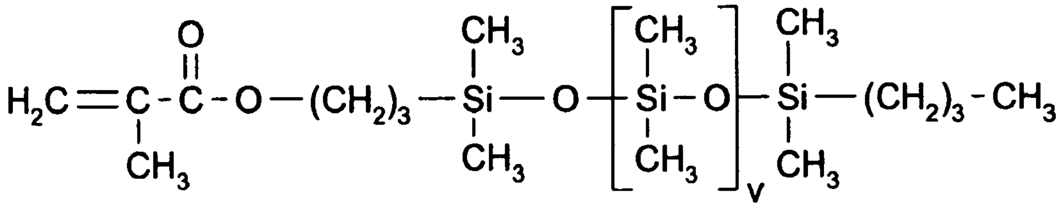

(XIII)

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :

- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;

- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :

- le "Croquat L" dont les groupements ammonium quaternaires comportent un groupement alkyle en $C_{12}$
- le "Croquat M" dont les groupements ammonium quatemairees comportent des groupements alkyle en $C_{10}$-$C_{18}$;
- le "Croquat S" dont les groupements ammonium quaternaires comportent un groupement alkyle en $C_{18}$ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule (XIV) :

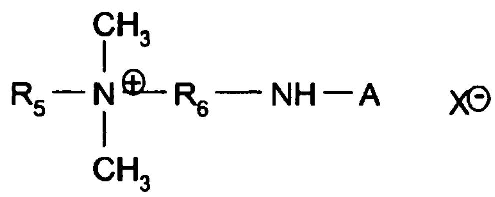

(XIV)

dans laquelle X- est un anion d'un acide organique ou minéral, A désigne un reste de protéine dérivé d'hydrolysats de protéine de collagène, $R_5$ désigne un groupement lipophile comportant jusqu'à 30 atomes de carbone, $R_6$ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quatemisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Selon la présente invention, Les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/1666, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl emino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique.
- le N-docosanoyl N-méthyl-D-glucamine

ou les mélanges de ces composés.

On peut également utiliser des tensioactifs cationiques parmi lesquels on peut citer en particulier : les sels

d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple :

- ceux qui présentent la formule générale (XV) suivante :

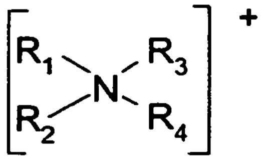

(XV)

dans laquelle les radicaux $R_1$ à $R_4$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone; X- est un anion choisi dans le groupe des halogénures, phosphates, acétates, lactates, alkyl($C_2$-$C_6$)sulfates, alkyl-ou-alkylarylsulfonates,

- les sels d'ammonium quaternaire de l'imidazolinium, comme par exemple celui de formule (XVI) suivante :

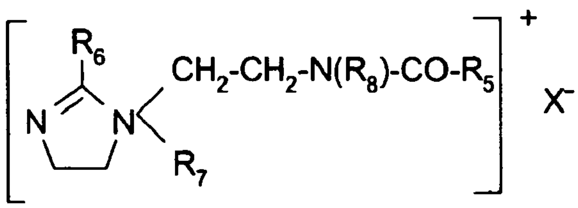

(XVI)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical aikyie en $C_1$-$C_4$, $R_8$ représente un atome d'hydrogène, un radical alkyle en $C_1$-$C_4$, X- est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple commercialisé sous la dénomination «REWOQUAT W 75» par la société DEGUSSA,

- les sels de diammonium quaternaire de formule (XVII) :

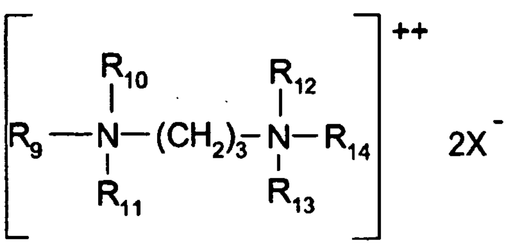

(XVII)

dans laquelle $R_9$ désigne un radical aliphatique comportant environ de 16 à 30 atomes de carbone, $R_{10}$, $R_{11}$, $R_{12}$, $R_{13}$ et $R_{14}$, identiques ou différents sont choisis parmi l'hydrogène ou un radical alkyle comportant de 1 à 4 atomes de carbone, et X- est un anion choisi dans le groupe des halogénure, acétates, phosphates, nitrates et méthylsulfates. De tels sels de diammonium quaternaire comprennent notamment le dichlorure de propanesuif diammonium.

- les sels d'ammonium quaternaire contenant au moins une fonction ester

Les sels d'ammonium quaternaire contenant au moins une fonction ester utilisables selon l'invention sont par exemple ceux de formule (XVIII) suivante :

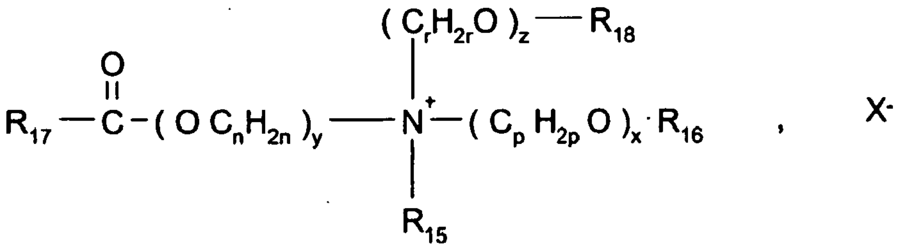

(XVIII)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical

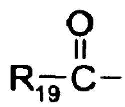

  - les radicaux $R_{20}$ hydrocarbonés en $C_1$-$C_{22}$ linéaires ou ramifiés, saturés ou insaturés,
  - atome d'hydrogène,
- $R_{18}$ est choisi parmi :
  - le radical

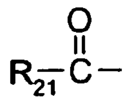

  - les radicaux $R_{22}$ hydrocarbonés en $C_1$-$C_6$ linéaires ou ramifiés, saturés ou insaturés,
  - l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

Les radicaux alkyles $R_{15}$ peuvent être linéaires ou ramifiés et plus particulièrement linéaires.

De préférence $R_{15}$ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque $R_{16}$ est un radical $R_{20}$ hydrocarboné, il peut être long et avoir de 12 à 22 atomes de carbone ou court et avoir de 1 à 3 atomes de carbone.

Lorsque $R_{18}$ est un radical $R_{22}$ hydrocarboné, il a de préférence 1 à 3 atomes de carbone.

Avantageusement, $R_{17}$, $R_{19}$ et $R_{21}$, identiques ou différents, sont choisis parmi les radicaux hydrocarbonés en $C_{11}$-$C_{21}$, linéaires ou ramifiés, saturés ou insaturés, et plus particulièrement parmi les radicaux alkyle et alcényle en $C_{11}$-$C_{21}$, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement les sels d'ammonium de formule (XVIII) dans laquelle :

- $R_{15}$ désigne un radical méthyle ou éthyle,
- x et y sont égaux à 1;
- z est égal à 0 ou 1 ;
- n, p et r sont égaux à 2 ;
- $R_{16}$ est choisi parmi :
  - le radical

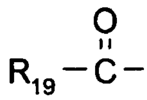

- les radicaux méthyle, éthyle ou hydrocarbonés en $C_{14}$-$C_{22}$
  - l'atome d'hydrogène ;
- $R_{18}$ est choisi parmi :
  - le radical

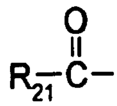

  - l'atome d'hydrogène ;

$R_{17}$, $R_{19}$ et $R_{21}$, identiques ou différents, sont choisis parmi les radicaux hydrocarbonés en $C_{13}$-$C_{17}$, linéaires ou ramifiés, saturés ou insaturés et de préférence parmi les radicaux alkyles et alcényle en $C_{13}$-$C_{17}$, linéaires ou ramifiés, saturés ou insaturés.
Avantageusement, les radicaux hydrocarbonés sont linéaires.

On peut citer par exemple les composés de formule (XVI) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme
ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quatemisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUATWE 18 par la société DEGUSSA.

On peut aussi utiliser les sels d'ammonium contenant au moins une fonction ester décrits dans les brevets US-A-4874554 et US-A-4137180.

Parmi les sels d'ammonium quaternaire de formule (XV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Les acides gras sont choisis plus particulièrement parmi l'acide myristique, l'acide palmitique, l'acide stéarique, l'acide béhénique, l'acide oléique, l'acide linoléïque, l'acide linolénique et l'acide isostéarique,

Les dérivés d'acides gras sont notamment les esters d'acides carboxyliques en particulier les esters mono, di, tri ou tétracarboxyliques.
Les esters d'acides monocarboxyliques sont notamment les monoesters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$ , le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, le néopentanoate d'isostéaryle, le néopentanoate d'isodécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicapryiate le dicaprate de propylène giycoi ; i'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle, le néopentanoate d'isostéaryle, le néopentanoate d'isodécyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents conditionneur.

Selon l'invention, le ou les agentsconditionneurs peuvent représenter de 0,001 % à 20% en poids, de préférence de 0,01 % à 10% en poids et plus particulièrement de 0,1 à 3% en poids par rapport au poids total de la composition finale.

Les compositions de l'invention peuvent contenir en outre avantageusement au moins un agent tensioactif qui est généralement présent en une quantité comprise entre 0,1% et 60% en poids environ, de préférence entre 1% et

40% et encore plus préférentiellement entre 5% et 30%, par rapport au poids total de la composition.

Cet agent tensioactif peut être choisi parmi les agents tensioactifs anioniques, cationiques, amphotères, non-ioniques ou des mélanges

Les tensioactifs convenant à la mise en oeuvre de la présente invention sont notamment les suivants :

(i) Tensioactif(s) anionique(s) :

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$ -oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements giycéroi pouvant aller notamment de 2 à 30. On peut également citer les copolymère d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaines ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{ -CONHCH}_2\text{CH}_2\text{ -N(R}_3\text{)(R}_4\text{)(CH}_2\text{COO-)} \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement

carboxyméthyte ;
et

$$R_5\text{-CONHCH}_2\text{CH}_2\text{-N(B)(C)} \quad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$- CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.
Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.
On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30° en solution aqueuse à 32 % de MA par la société COGNIS.

La composition de l'invention peut également contenir au moins un additif choisi parmi les épaississants minéraux ou organiques, associatifs ou non (en particulier des polyuréthannes polyéthers associatifs cationiques ou non ioniques), les parfums, les agents nacrants, les conservateurs, les polymères anioniques ou non ioniques, les protéines non cationiques, les hydrolysats de protéines non cationiques, l'acide méthyl-18 eicosanoique, les hydroxyacides et tout autre additif classiquement utilisé dans le domaine cosmétique qui n'affecte pas les propriétés des compositions selon l'invention.
Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.
Les compositions conformes à l'invention peuvent être plus particulièrement utilisées pour le lavage ou le traitement des fibres kératiniques telles que les cheveux.
Les compositions selon l'invention peuvent être des shampooings. Dans ce mode de réalisation de l'invention, les compositions comprennent une base lavante, généralement aqueuse.
Le ou les tensioactifs formant la base lavante peuvent être indifféremment choisis, seuls ou en mélanges, au sein des tensioactifs anioniques, amphotères, non ioniques tels que définis ci-dessus. La quantité et la qualité de la base lavante sont celles suffisantes pour conférer à la composition un pouvoir moussant et/ou détergent satisfaisant. Ainsi, selon l'invention, la base lavante peut représenter de 4 % à 50 % en poids, de préférence de 6 % à 35 % en poids, et encore plus préférentiellement de 8 % à 25 % en poids, du poids total de la composition. De préférence, la base lavante contient au moins 3% en poids de tensioactif anioniques, plus particulièrement de 4 à 30% en poids par rapport au poids total de la composition.
Les compositions de l'invention peuvent également se présenter sous forme d'après-shampooing à rincer ou non ; ou encore sous forme de composition à appliquer après une coloration, une décoloration, une permanente ou un défrisage ou encore entre les deux étapes d'une permanente ou d'un défrisage.

Les compositions selon l'invention peuvent également se présenter sous forme de lotions aqueuses ou hydroalcooliques pour le soin des cheveux. Les compositions cosmétiques selon l'invention peuvent également se présenter sous forme de gel, de lait, de crème, d'émulsion ou de mousse et être utilisées les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Le pH de la composition appliquée sur les fibres kératiniques varie généralement de 1 à 11. Il est de préférence de 2 à 6, et peut être ajusté à la valeur désirée au moyen d'agents acidifiants ou alcalinisants bien connus de l'état de la technique des compositions appliquées sur des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XX) suivante :

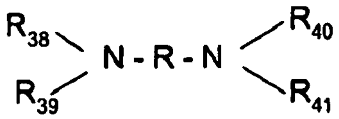
(XX)

dans laquelle R est un reste propylène éventuellement substitué par un groupement hydroxyle ou un radical alkyle en $C_1$-$C_4$ ; $R_{38}$, $R_{39}$, $R_{40}$ et $R_{41}$, identiques ou différents, représentent un atome d'hydrogène, un radical alkyle en $C_1$-$C_4$ ou hydroxyalkyle en $C_1$-$C_4$-

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

L'invention a aussi pour objet un procédé de traitement des fibres kératiniques telles que les cheveux, caractérisé en ce qu'il consiste à appliquer sur les matières kératiniques une composition cosmétique telle que définie précédemment, puis à effectuer éventuellement un rinçage à l'eau.

Selon un mode particulier de réalisation, les compositions selon l'invention seront utilisées comme composition de traitement des fibres kératiniques en particulier des cheveux teints par coloration directe ou par coloration d'oxydation.

Un objet de l'invention consiste donc en l'utilisation en post traitement d'une coloration d'oxydation ou d'une coloration directe des fibres kératiniques et plus particulièrement des cheveux d'une composition telle que définie précédemment

Un autre objet de l'invention concerne un procédé de coloration consistant à appliquer sur les fibres kératiniques humaines et plus particulièrement des cheveux, une composition (A) colorante directe ou d'oxydation pendant un temps suffisant pour développer la couleur, et de faire suivre cette application, après rinçage ou non rinçage, séchage ou non séchage, par l'application d'une composition (B) constituée par une composition selon l'invention telle que définie précédemment..

Comme il est indiqué ci dessus, la composition de post traitement selon l'invention peut être appliquée immédiatement après coloration ou de manière différée. Par différée, on entend une application se faisant quelques heures, un jour ou plusieurs jours (de 1 à 60 jours) après la coloration.

De préférence, plusieurs applications de la composition de l'invention sont effectuées entre deux colorations.

Le nombre d'applications entre deux colorations est de préférence compris entre 1 et 60 et encore plus préférentiellement entre 2 et 30.

La composition de post traitement peut être utilisée en mode rincé ou en mode non-rincé, c'est à dire que son application est suivie ou non d'un rinçage.

Dans le premiercas, le temps de pose de la composition de posttraitement est compris entre quelques secondes et 90 minutes et de préférence entre 30 secondes et 20 minutes.

La température d'application de la composition de post traitement peut varier de 10°C à 70°C. De préférence l'application s'effectuera entre 10 et 60°C et plus particulièrement à la température ambiante.

La nature et la concentration des colorants présents dans les compositions colorantes n'est pas critique.

Dans le cas des colorations directes éclaircissantes les compositions colorantes (A) résultent du mélange au moment de l'emploi d'une composition colorante (A1) contenant au moins un colorant direct et d'une composition (A2) contenant un agent oxydant.

Dans le cas des colorations d'oxydation, les compositions colorantes (A) résultent du mélange au moment de l'emploi d'une composition colorante (A1) contenant au moins une base d'oxydation et éventuellement au moins un coupleur et/ou un colorant direct et d'une composition (A2) contenant un agent antioxydant.

Les colorants directs sont plus particulièrement des composés absorbant les radiations lumineuses dans le domaine visible (400-750 nm). Ils peuvent être de nature non ionique, anionique ou cationique.

Généralement, les colorants directs sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ceux dérivés du triarylméthane, seuls ou en mélanges.

Parmi les colorants benzéniques nitrés, on peut citer les composés rouges ou orangés suivants : le 1-hydroxy-3-nitro-4-N-(γ-hydroxypropyl)amino benzène, le N-(β-hydroxy éthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)-amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxy éthyl)amino-5-chloro benzène, la 2-nitro-4-amino-diphénylamine, le 1-amino-3-nitro-6-hydroxybenzène, le 1-(β-amino éthyl)amino-2-nitro-4-(β-hydroxy éthyloxy) benzène, le 1-(β,γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β -hydroxyéthyl)amino benzène, la 2-nitro-4'-hydroxydiphénylamine, le 1-amino-2-nitro-4-hydroxy-5-méthylbenzène, seuls ou en mélanges.

En ce qui concerne les colorants directs benzéniques nitrés, on peut mettre en oeuvre des colorants de ce type jaunes et jaune-verts, comme par exemple le 1-β-hydroxyéthyloxy-3-méthylamino-4-nitrobenzène, le 1-méthylamino-2-nitro-5-(β,γ- dihydroxypropyl)oxy benzène, le 1-(β-hydroxyéthyl)amino-2-méthoxy-4-nitrobenzène, le 1-(β-amino éthyl)amino-2-nitro-5-méthoxy-benzène, le 1,3-di(β-hydroxyéthyl)amino-4-nitro-6-chloro benzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluoro-méthylaniline, l'acide 4-(β-hydroxy éthyl)amino-3-nitro-benzènesulfonique, l'acide 4-éthylamino-3-nitro-benzoïque, le 4-(β -hydroxyéthyl)amino-3-nitro-chlorobenzène, le 4-(β-hydroxyéthyl)amino-3-nitro-méthyl benzène, le 4-(β,γ-dihydroxypropyl)amino-3-nitro-trifluorométhylbenzène, le 1-(β-uréido éthyl)amino-4-nitrobenzène, le 1,3-diamino-4-nitrobenzène, le 1-hydroxy-2-amino-5-nitrobenzène, le 1-amino-2-[tris(hydroxyméthyl)méthyl]amino-5-nitro-benzène, le 1-(β-hydroxyéthyl)amino-2-nitrobenzène, le 4-(β-hydroxyéthyl)amino-3-nitrobenzamide.

Il est de même envisageable d'utiliser des colorants benzéniques nitrés bleus ou violets, comme entre autres le 1-(β-hydroxyéthyl)amino-4-N,N-bis-(β-hydroxyéthyl)amino 2-nitrobenzène, le 1-(γ-hydroxypropyl)amino 4-N,N-bis-(β-hydroxyéthyl)amino 2-nitrobenzène, le 1-(β-hydroxyéthyl)amino 4-(N-méthyl, N-β-hydroxyéthyl)amino 2-nitrobenzène, le 1-(β-hydroxyéthyl)amino4-(N-éthyl, N- β-hydroxyéthyl)amino 2-nitrobenzène, le 1-(β,γ-dihydroxypropyl) amino 4-(N-éthyl, N-β-hydroxyéthyl)amino 2-nitrobenzène, les 2-nitroparaphénylènediamines de formule suivante :

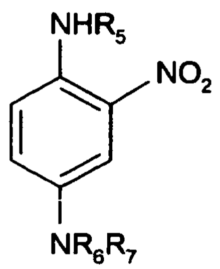

dans laquelle :

- $R_6$ représente un radical alkyle en $C_1$-$C_4$, un radical β-hydroxyéthyle ou β-hydroxypropyle ou γ-hydroxypropyle ;
- $R_5$ et $R_7$, identiques ou différents, représentent un radical β-hydroxyéthyle β-hydroxypropyle, γ-hydroxypropyle, ou β, γ-dihydroxypropyle, l'un au moins des radicaux $R_6$, $R_7$ ou $R_5$ représentant un radical γ-hydroxypropyle et $R_6$ et $R_7$ ne pouvant désigner simultanément un radical β-hydroxyéthyle lorsque $R_6$ est un radical γ-hydroxypropyle, telles que celles décrits dans le brevet français FR 2 692 572.

Il est rappelé que les colorants azoïques sont des composés comportant dans leur structure au moins un enchaînement -N=N- non inclus dans un cycle ; les colorants méthiniques sont des composés comportant dans leur structure au moins un enchaînement -C=C- non inclus dans un cycle ; les colorants azométhinique sont des composés comportant dans leur structure au moins un enchaînement -C=N- non inclus dans un cycle.

Les colorants dérivés de triarylméthane comportent dans leur structure au moins un enchaînement suivant :

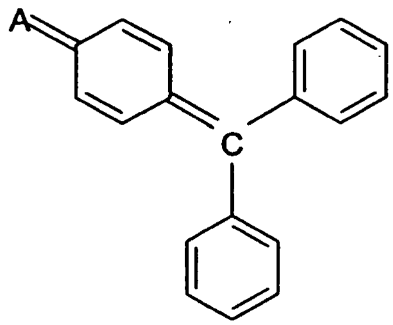

A désignant un atome d'oxygène ou d'azote

Les colorants xanthéniques comportent dans leur structure au moins un enchaînement de formule :

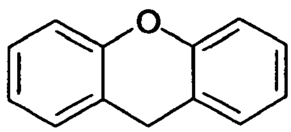

Les colorants phénanthridiniques comportent dans leur structure au moins un enchaînement de formule

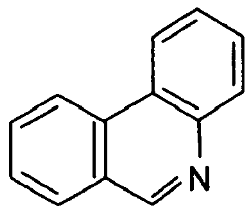

Les colorants phtalocyanines comportent dans leur structure au moins un enchaînement de formule :

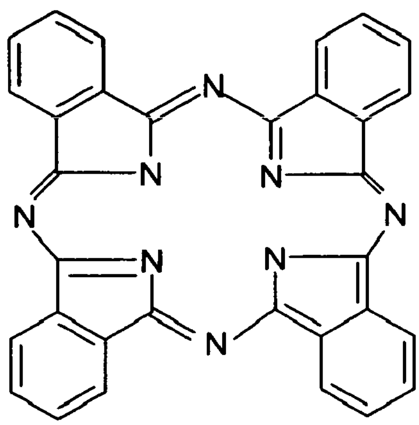

Les colorants phénotiaziniques comportent dans leur structure au moins un enchaînement suivant :

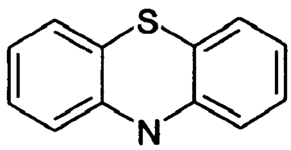

Les colorants directs peuvent de plus être choisis parmi les colorants basiques comme ceux listés dans le Color Index, 3ème édition, notamment sous les dénominations "Basic Brown 16", "Basic Brown 17", "Basic Yellow 57",

"Basic Red 76", "Basic Violet 10", "Basic Blue 26" et "Basic Blue 99" ; ou parmi les colorants directs acides, listés le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore les colorants directs cationiques tels que ceux décrits dans les demandes de brevet WO 95/01772, WO 95/15144 et EP 714954 et dont le contenu fait partie intégrante de la présente invention.

Lorsqu'ils sont présents, le ou les colorants directs représentent de préférence de 0,0005 à 12 % en poids environ du poids total de la composition tinctoriale, et encore plus préférentiellement de 0,005 à 6 % en poids environ de ce poids.

Les bases d'oxydation peuvent être choisies parmi les bases d'oxydation classiquement utilisées en teinture d'oxydation et parmi lesquelles on peut notamment citer les paraphénylènediamines , les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-p-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 6-hydroxybenzomorpholine, la 3,5 diamino 2,6-diméthoxy pyridine, le 1-N-(βhydroxyéthyl)amino 3,4 méthylènedioxy benzène, le 2,6 bits(β hydroxyéthylamino)toluène, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La nature de l'agent oxydant utilisé dans la coloration directe éclaircissante (coloration directe avec un agent oxydant) ou dans la coloration d'oxydation n'est pas critique.

L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

L'invention a également pour objet un agent de coloration multi-composants ou kit comportant au moins un premier composant comprenant une composition (A) de coloration directe et un deuxième composant comprenant une composition (B) de post-traitement constituée d'une composition telle que définie précédemment.

L'invention a également pour objet un agent de coloration multi-composants ou kit comportant au moins un premier composant comprenant une composition (A1) comprenant au moins un colorant direct, un deuxième composant

comprenant une composition (A2) contenant au moins un agent oxydant et un troisième composant comprenant une composition (B) de post-traitement constituée d'une composition telle que définie précédemment.

L'invention a également pour objet un agent de coloration multi-composants ou kit comportant au moins un premier composant comprenant une composition (A1) contenant au moins un précurseur de colorant d'oxydation, un deuxième composant comprenant une composition (A2) contenant au moins un agent oxydant et un troisième composant comprenant une composition (B) de post-traitement constituée d'une composition telle que définie précédemment.

Les exemples suivants sont destinés à illustrer l'invention. Celle-ci n'est cependant pas limitée à ces modes de réalisation.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

**EXEMPLES:**

On a réalisé les essais suivants :

On a coloré des cheveux permanentés en bleu à partir d'une composition de teinture détaillée dans le tableau 1 ci-dessous contenant une base de coloration d'oxydation : la para-phénylène diamine en association équimolaire avec un coupleur de coloration d'oxydation : le 2,4 diamino phenoxyethanol

**Tableau 1**

| Composition colorante | Quantités |
|---|---|
| Hydroxyethylcellulose | 0.768 g% |
| Decyl Glucoside | 5.4 g% |
| PEG-1412 | 2.7 g% |
| Alcool Benzylique | 1.8 g% |
| Ethanol | 18 g% |
| Para-phénylène diamine | $7.5.10^{-4}$ mol% |
| 2,4 diamino phenoxyethanol | $7.5.10^{-4}$ mol% |
| Dissolvine | 1.08 g% |
| Métabisulfite de sodium | 0.205 g% |
| Ammoniaque à 20% en ammoniac | 10 g% |
| Eau déminéralisée | Qsp100 g% |

Au moment de l'emploi, la composition colorante d'oxydation ci-dessus est mélangée poids pour poids avec de l'eau oxygénée (Eau Oxygénée l'Oréal Professionnel 20 volumes à 6% de peroxyde d'hydrogène).

Le mélange est ensuite appliqué sur des mèches de cheveux permanentées, à raison de 10 g de mélange colorant/g de mèche. Le temps de pause est de 15 mn de chaque côté de la mèche.

La coloration est ensuite stoppée par un rinçage à l'eau suivi d'un lavage avec un shampooing commercial (shampooing DOP camomille). Puis les mèches sont séchées 30 minutes à 60°C au casque.

**Post-traitements photo-protecteurs :**

On applique alors, 24 heures après la coloration décrite ci-dessus, les compositions rassemblées dans le tableau 2 sur les mèches colorées.

**Tableau 2**

| **Ingrédients** | **Composition 1 (invention)** | **Composition 2 (hors invention)** |
|---|---|---|
| Ethanol | 15 g% | 15 g% |
| Alcool benzylique | 5g% | 5g% |
| Acide benzoïque | 0.2g% | - |
| Acide citrique | - | 0.2g% |
| Benzophenone-4, commercialisée sous la dénomination Uvinul MS40 par BASF | 20g%ma | 20g%ma |
| Eau déminéralisée | Qsp100 | Qsp100 |
| aspect | limpide | limpide |

Pour cela, les mèches colorées sont traitées avec les compositions ci dessus. Le temps de contact est de 10 minutes à une température de 45°C.

Le traitement est suivi d'un shampooing d'élimination avec un shampooing commercial (shampooing DOP camomille). Puis les mèches sont séchées 30 minutes à 60°C au casque.

**Exposition UV**

Les mèches colorées et traitées sont alors exposées aux UV sur la moitié de leur longueur pendant une période de 40H par un simulateur solaire le Xénotest 150S L'autre moitié de la mèche est masquée par un papier cartonné.

Le Xénotest 150S reproduit un spectre lumineux reproductible et proche de celui du soleil par l'émission d'une lampe à arc au xénon XE 1501 filtrée par 6 filtres Infrarouge et un filtre UV (UG11-noir). Les mèches en rotation face à ce rayonnement reçoivent alors une énergie de 1250 W/$m^2$ dans un domaine spectral de 300 à 800 nm (UV+visible) dans des conditions de température de 30°C, et d'Humidité Relative de 51.6%.

**Evaluation de la photo-protection**

La dégradation de la couleur après exposition UV est évaluée colorimétriquement entre les zones de mèches masquées et non masquées.

Les mesures sont réalisées à l'aide du spectro-colorimétrique MINOLTA CM2022 24 heures après coloration et 24 heures après les 40H d'exposition UV.

La dégradation provoquée par les UV est exprimée en ΔE

$$\Delta E^2 = (\Delta L^*_{\text{après exposition}} - \Delta L^*_{\text{avant exposition}})^2 + (\Delta a^*_{\text{après exposition}} - \Delta a^*_{\text{avant exposition}})^2 + (\Delta b^*_{\text{après exposition}} - \Delta b^*_{\text{avant exposition}})^2$$

Les résultats obtenus sont indiqués dans le tableau 3 ci-dessous :

Tableau 3

| **Composition** | ΔE |
|---|---|
| Témoin non traité | 11.47 |
| Compo 1 (invention) | 5.07 |
| Compo 2 (hors invention) | 7.6 |

On observe que la composition 1 selon l'invention contenant l'acide benzoïque présente une meilleure résistance à la lumière de la couleur des cheveux après 40H d'exposition UV que celle obtenue avec la composition 2 ne contenant pas d'acide benzoïque.

## Revendications

1. Composition cosmétique comprenant, dans un milieu aqueux physiologiquement acceptable :

    a) au moins un agent protecteur des fibres kératiniques choisi parmi les filtres UV organiques dans une quantité allant de 0,2 à 20% en poids par rapport au poids total de la composition ;
    b) au moins un alcool aromatique qui est un composé liquide à température ambiante et pression atmosphérique comprenant au moins un cycle benzénique ou mephtalémique et au moins une fonction alcool (OH) sur au moins un substituant dudit cycle dans une quantité supérieure à 1% en poids par rapport au poids total de la composition ; et
    c) au moins un acide carboxylique aromatique sont choisis parmi
    l'acide benzoïque
    l'acide salicylique
    ainsi que leurs formes salifiées et leurs mélanges.

2. Composition selon la revendication 1, où le milieu aqueux est constitué par au moins de l'eau et contient au moins un solvant organique différent des alcools aromatiques et des acides carboxyliques aromatiques.

3. Composition selon la revendication 2, où ledit solvant organique additionnel est choisi parmi les alcools inférieurs en $C_1$-$C_4$ et les polyols.

4. Composition selon la revendication 2 ou 3, où ledit solvant organique est l'éthanol.

5. Composition selon l'une quelconque des revendications 1 à 4, où les filtres UV organiques sont choisis parmi les filtres hydrosolubles ou liposolubles, siliconés ou non siliconés.

6. Composition selon la revendication 5, où les filtres UV organiques sont choisis parmi les dérivés de dibenzoylméthane ; les anthranilates ; les dérivés cinnamiques : les dérivés salicyliques, les dérivés du camphre ; les dérivés de la benzophénone ; les dérivés de β,β-diphénylacrylate ; les dérivés de trlazlne ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de benzoxazolen ; les polymères filtres et silicones filtres; les dimères dérivés d'α-alkylstyrène; les 4,4-darylbutadiènes et leurs mélanges.

7. Composition selon la revendication 5 ou 6, où les filtres UV organiques liposolubles sont choisis parmi :

    Butyl Methoxydibenzoylmethane
    Ethylhexyl Methoxycinnamate
    Homosalate
    Ethylhexyl Salicylate,
    Octoayene,
    Benzophenone-3,
    2-(4-diethylamino-2-hydroxybenzoyl)-benzoate de n-hexyle-
    4-Methymenzylidene camphor,
    Ethylhexyl triazone,
    Bis-Ethylhexyloxyphenol Methoxyphenyl Triazine
    Diethylhexyl Butamido Triazone,
    Drometrizole Trisiloxane
    Polysilicone-15
    1,1-dicarboxy (2,2'-diméthyl-propyl)-4,4-diphénybutadiène
    2,4-bis-[5-1(diméthylpropyl)benzoxazol-2-yl-(4-phenyl)-imino]-6-(2-ethylhexyl)-imino-1,3,5-triazine

    ou leurs mélanges.

8. Composition selon la revendication 6 ou 7, où les filtres UV organiques hydrosolubles sont choisis parmi :

    PABA,
    PEG-25 PABA
    Benzylidene Camphor Sulfonic Acid,

Camphor Benzalkonium Methosulfate
Terephthalylidene Dicamphor Sulfonic Acid
Phenylbenzimidazole Sulfonic Acid
Disodium Phenyl Dibenzimidazole Tetra-sulfonate
Benzophenone-4
Benzophanone-5

**9.** Composition selon l'une quelconque des revendications 1 à 8, dans laquelle le ou les agents protecteurs sont solubles dans le milieu aqueux de la composition.

**10.** Composition selon la revendication 9, où le ou les agents protecteurs ont un log P (coefficient de partage d'octanol/eau) inférieur à 9 et plus préférentiellement inférieur à 4.

**11.** Composition selon la revendication 9 ou 10, dans laquelle le ou les agents protecteurs sont solubles à au moins 0,5% dans l'eau ou les alcools inférieurs en $C_I$-$C_4$ comme l'éthanol à 25°C.

**12.** Composition selon l'une quelconque des revendications 1 à 11, dans laquelle le ou les agents protecteurs sont des filtres UV organiques hydrosolubles.

**13.** Composition selon la revendication 12, où le filtre UV hydrosoluble est la Benzophénone-4.

**14.** Composition selon l'une quelconque des revendications 1 à 13, dans laquelle l'alcool aromatique est choisi parmi

l'alcool benzylique
le benzoyl isopropanol
le benzylglycol
le phénoxyéthanol
l'alcool di-chloro-benzylique
le méthylphenylbutanol
le phenoxyisopropanol
le phénylisohexanol
le phénylpropanol
l'alcool phényléthylique

et leurs mélanges.

**15.** Composition selon la revendication 14, où l'alcool aromatique est l'alcool benzylique.

**16.** Composition selon la revendication 15, dans laquelle l'acide carboxylique aromatique est l'acide benzoïque.

**17.** Composition selon l'une quelconque des revendications 1 à 16, dans laquelle le ou les acides carboxyliques aromatiques ou leurs sels représentent de 0,001 % à 30 % en poids, de préférence de 0,01 % à 20% en poids et plus particulièrement de 0,1 à 10% en poids par rapport au poids total de la composition.

**18.** Composition selon l'une quelconque des revendications 1 à 17, contenant en plus un ou plusieurs agents de conditionnement.

**19.** Composition selon la revendication 18, où le ou les agents conditionneurs représentent de 0,001 % à 20 % en poids, de préférence de 0,01 % à 10% en poids et plus particulièrement de 0,1 à 3% en poids par rapport au poids total de la composition.

**20.** Composition selon l'une quelconque des revendications 1 à 19, contenant en plus un ou plusieurs agents tensioactifs anioniques, cationiques, amphotères ou non-ioniques.

**21.** Composition selon la revendication 20, où le ou les agents tensioactifs sont présents en une quantité comprise entre 0,1% et 60% en poids environ, de préférence entre 1% et 40% et encore plus préférentiellement entre 5% et 30%, par rapport au poids total de la composition.

**22.** Composition selon l'une quelconque des revendications 1 à 21, **caractérisée par le fait qu'**elle se présente sous forme de shampooing et comprend une base lavante.

**23.** Composition selon la revendication 22, dans laquelle le ou les tensioactifs formant la base lavante représentent de 4 % à 50 % en poids, de préférence de 6 % à 35 % en poids, et encore plus préférentiellement de 8 % à 25 % en poids, du poids total de la composition.

**24.** composition selon la revendication 23, contenant au moins 3% en poids de tensioactif anionique, plus particulièrement de 4 à 30% en poids par rapport au poids total de la composition.

**25.** Composition selon l'une quelconque des revendications précédentes, **caractérisée par le fait qu'**elle se présente sous forme de lotion aqueuse ou hydroalcoolique ;sous forme de gel, de lait, de crème, d'émulsion, de mousse.

**26.** Composition selon l'une quelconque des revendications précédentes, **caractérisée par le fait qu'**elle est conditionnée sous forme de vaporisateur, de flacon pompe ou dans des récipient aérosol.

**27.** Composition selon l'une quelconque des revendications précédentes, **caractérisée par le fait qu'**elle se présente sous forme d'après-shampooing à rincer ou non ; sous forme de composition à appliquer après une coloration, une décoloration, une permanente ou un défrisage ou encore entre les deux étapes d'une permanente ou d'un défrisage.

**28.** Composition selon l'une quelconque des revendications 1 à 27, **caractérisée par le fait que** le pH de la composition appliquée sur les fibres kératiniques varie de 1 à 11.

**29.** Composition selon la revendication 28, où le pH varie de 2 à 6.

**30.** Utilisation d'une composition telle que définie dans l'une quelconque des revendications précédentes dans la préparation d'une formulation cosmétique destinée à la protection des fibres kératiniques contre l'action des agents atmosphériques.

**31.** Utilisation d'une composition telle que définie dans l'une quelconque des revendications précédentes comme formulation de post-traitement des fibres kératiniques teintes en particulier des cheveux teints par coloration directe ou par coloration d'oxydation.

**32.** Procédé de coloration des fibres kératiniques et plus particulièrement des cheveux, consistant à appliquer sur lesdites fibres, une composition (A) colorante directe ou d'oxydation pendant un temps suffisant pour développer la couleur, et de fairesuivre cette application, après rinçage ou non rinçage, séchageou non séchage, par l'application d'une composition (B) constituée d'une composition selon l'une quelconque des revendications précédentes ; ladite composition (B) étant appliquée, soit immédiatement, soit en différé, et les applications de ladite composition pouvant être répétées entre deux colorations.

**33.** Agent de coloration multi-composants comportant au moins un premier composant comprenant une composition (A) colorante directe ou d'oxydation et un deuxième composant comprenant une composition (B) de post-traitement constituée d'une composition selon l'une quelconque des revendications précédentes.

**34.** Agent de coloration multi-composants comportant au moins un premier composant comprenant une composition ($A_1$) comprenant au moins un colorant direct, un deuxième composant comprenant une composition ($A_2$) contenant au moins un agent oxydant et un troisième composant comprenant une composition (B) de post-traitementconstituée d'une composition selon l'une quelconque des revendications précédentes.

**35.** Agent de coloration multi-composants comportant au moins un premier composant comprenant une composition (A,) contenant au moins un précurseur de colorant d'oxydation, un deuxième composant comprenant une composition ($A_2$) contenant au moins un agent oxydant et un troisième composant comprenant une composition (B) de post-traitement constituée d'une composition selon l'une quelconque des revendications précédentes

## Patentansprüche

**1.** Kosmetische Zusammensetzung, die in einem physiologisch akzeptablen wässerigen Medium enthält:

a) mindestens ein Schutzmittel für Keratinfasern, das unter organischen UV-Filtern ausgewählt ist, in einer Menge von 0,2 bis 20 Gew. -%, bezogen auf das Gesamtgewicht der Zusammensetzung;
b) mindestens einen aromatischen Alkohol, der bei Umgebungstemperatur und Normaldruck eine flüssige Verbindung ist, die mindestens einen Benzol- oder Naphthalinring und mindestens eine Alkoholfunktion (OH) an mindestens einem Substituenten des Rings umfasst, in einer Menge von mehr als 1 Gew.-%, bezogen auf das Gesamtgewicht der Zusammensetzung; und
c) mindestens eine aromatische Carbonsäure, die ausgewählt ist unter

Benzoesäure, Salicylsäure sowie ihren Salzformen und Gemischen dieser Verbindungen.

**2.** Zusammensetzung nach Anspruch 1, bei der das wässerige Medium zumindest aus Wasser besteht und mindestens ein organisches Lösungsmittel enthält, das von den aromatischen Alkoholen und den aromatischen Carbonsäuren verschieden ist.

**3.** Zusammensetzung nach Anspruch 2, bei der das zusätzliche organische Lösungsmittel unter niederen $C_1$-$C_4$-Alkoholen und Polyolen ausgewählt ist.

**4.** Zusammensetzung nach Anspruch 2 oder 3, bei der das organische Lösungsmittel Ethanol ist.

**5.** Zusammensetzung nach einem der Ansprüche 1 bis 4, bei der die UV-Filter unter wasserlöslichen oder fettlöslichen, siliconhaltigen oder nicht siliconhaltigen Filtern ausgewählt sind.

**6.** Zusammensetzung nach Anspruch 5, bei der die organischen UV-Filter ausgewählt sind unter Derivaten von Dibenzoylmethan; Anthranilaten, Zimtsäurederivaten; Salicylsäurederivaten; Campherderivaten; Benzophenonderivaten; Derivaten von $\beta,\beta$-Diphenylacrylat; Triazinderivaten; Benzotriazolderivaten; Benzalmalonatderivaten; Benzimidazolderivaten; Imidazolinen; Bis-benzoazolylderivaten; Derivaten von p-Aminobenzoesäure (PABA); Benzoxazolderivaten; polymeren Filtern und Siliconfiltern; von $\alpha$-Alkylstyrolen abgeleiteten Dimeren und 4,4-Diarylbutadienen sowie Gemischen dieser Verbindungen.

**7.** Zusammensetzung nach Anspruch 5 oder 6, bei der die öllöslichen organischen UV-Filter ausgewählt sind unter
Butyl Methoxydibenzoylmethane,
Ethylhexyl Methoxycinnamate,
Homosalate,
Ethylhexylsalicylat,
Octocrylene,
Benzophenone-3,
2-(4-Diethylamino-2-hydroxybenzoyl)-benzoesäure-N-hexylester,
4-Methylbenzylidencampher,
Ethylhexyl Triazone,
Bis-Ethylhexyloxyphenol Methoxyphenyl Triazine Diethylhexyl Butamido Triazone,
Drometrizole Trisiloxane,
Polysilicone-15,
1,1-Dicarboxy-(2,2'-dimethylpropyl)-4,4-diphenylbutadien und
2,4-Bis[5-1(dimethylpropyl)-benzoxazol-2-yl-(4-phenyl)-imino]-6-(2-ethylhexyl)-imino-1,3,5-triazin sowie den Gemischen dieser Verbindungen.

**8.** Zusammensetzung nach Anspruch 6 oder 7, bei der die wasserlöslichen organischen UV-Filter ausgewählt sind unter
PABA,
PEG-25 PABA,
Benzylidene Camphor Sulfonic Acid,
Camphor Benzalkonium Methosulfate,
Terephthalylidene Dicamphor Sulfonic Acid,
Phenylbenzimidazole Sulfonic Acid,
Disodium Phenyl Dibenzimidazole Tetrasulfonate,
Benzophenone-4,
Benzophenone-5.

**9.** Zusammensetzung nach einem der Ansprüche 1 bis 8, bei der das Schutzmittel oder die Schutzmittel im wässerigen

Medium der Zusammensetzung löslich sind.

**10.** Zusammensetzung nach Anspruch 9, bei der das Schutzmittel oder die Schutzmittel einen Wert log P (Verteilungskoeffizient Octanol/Wasser) von weniger als 9 und noch bevorzugter von weniger als 4 besitzen.

**11.** Zusammensetzung nach Anspruch 9 oder 10, bei der das Schutzmittel oder die Schutzmittel in Wasser oder den niederen $C_1$-$C_4$-Alkoholen, wie Ethanol, bei 25 °C zu mindestens 0,5 % löslich sind.

**12.** Zusammensetzung nach einem der Ansprüche 1 bis 11, bei der das Schutzmittel oder die Schutzmittel wasserlösliche organische UV-Filter sind.

**13.** Zusammensetzung nach Anspruch 12, bei der das wasserlösliche UV-Filter Benzophenone-4 ist.

**14.** Zusammensetzung nach einem der Ansprüche 1 bis 13, bei welcher der aromatische Alkohol ausgewählt ist unter
Benzylalkohol,
Benzoylisopropanol,
Benzylglycol,
Phenoxyethanol,
Dichlorbenzylalkohol,
Methylphenylbutanol,
Phenoxyisopropanol,
Phenylisohexanol,
Phenylpropanol und
Phenylethylalkohol
sowie den Gemischen dieser Verbindungen.

**15.** Zusammensetzung nach Anspruch 14, bei welcher der aromatische Alkohol Benzylalkohol ist.

**16.** Zusammensetzung nach Anspruch 15, bei der die aromatische Carbonsäure Benzoesäure ist.

**17.** Zusammensetzung nach einem der Ansprüche 1 bis 16, bei der die aromatische Carbonsäure oder die aromatischen Carbonsäuren oder ihre Salze in einem Mengenanteil von 0,001 bis 30 Gew.-%, bevorzugt von 0,01 bis 20 Gew.-% und ganz besonders von 0,1 bis 10 Gew.-% vorliegen, bezogen auf das Gesamtgewicht der Zusammensetzung.

**18.** Zusammensetzung nach einem der Ansprüche 1 bis 17, die ferner ein oder mehrere Konditioniermittel enthält.

**19.** Zusammensetzung nach Anspruch 18, bei der das Konditioniermittel oder die Konditioniermittel in einem Mengenanteil von 0,001 bis 20 Gew.-%, vorzugsweise von 0,01 bis 10 Gew.-% und ganz besonders von 0,1 bis 3 Gew.-% vorliegen, bezogen auf das Gesamtgewicht der Zusammensetzung.

**20.** Zusammensetzung nach einem der Ansprüche 1 bis 19, die ferner ein oder mehrere anionische, kationische, amphotere oder nichtionische Tenside enthält.

**21.** Zusammensetzung nach Anspruch 20, bei der das Tensid oder die Tenside in einem Mengenanteil von etwa 0,1 bis 60 Gew.-%, bevorzugt von 1 bis 40 Gew.-% und noch bevorzugter von 5 bis 30 Gew.-% vorliegen, bezogen auf das Gesamtgewicht der Zusammensetzung.

**22.** Zusammensetzung nach einem der Ansprüche 1 bis 21, **dadurch gekennzeichnet, dass** sie in Form eines Shampoos vorliegt und eine Waschgrundlage enthält.

**23.** Zusammensetzung nach Anspruch 22, bei der das Tensid oder die Tenside, welche die Waschgrundlage bilden, in einem Mengenanteil von 4 bis 50 Gew.-%, bevorzugt von 6 bis 35 Gew.-% und noch bevorzugter von 8 bis 25 Gew.-% vorliegen, bezogen auf das Gesamtgewicht der Zusammensetzung.

**24.** Zusammensetzung nach Anspruch 23, die anionisches Tensid in einem Mengenanteil von mindestens 3 Gew.-% und insbesondere von 4 bis 30 Gew.-% enthält, bezogen auf das Gesamtgewicht der Zusammensetzung.

**25.** Zusammensetzung nach einem der vorhergehenden Ansprüche, **dadurch gekennzeichnet, dass** sie in Form einer

wässerigen oder wässerig-alkoholischen Lotion, in Form eines Gels, einer Milch, einer Creme, einer Emulsion oder eines Schaums vorliegt.

**26.** Zusammensetzung nach einem der vorhergehenden Ansprüche, **dadurch gekennzeichnet, dass** sie in Form eines Zerstäubers, eines Pumpflakons oder in einem Aerosolbehälter konfektioniert ist.

**27.** Zusammensetzung nach einem der vorhergehenden Ansprüche, **dadurch gekennzeichnet, dass** sie in Form eines auszuspülenden oder nicht auszuspülenden und nach dem Shampoonieren anzuwendenden Conditioners oder in Form einer nach einem Färben, einem Entfärben, einer Dauerwelle oder einem Entkräuseln oder auch zwischen den beiden Stufen einer Dauerwelle oder einer Entkräuselung anzuwendenden Zusammensetzung vorliegt.

**28.** Zusammensetzung nach einem der Ansprüche 1 bis 27, **dadurch gekennzeichnet, dass** der pH-Wert der auf die Keratinfasern aufgebrachten Zusammensetzung im Bereich von 1 bis 11 liegt.

**29.** Zusammensetzung nach Anspruch 28, bei welcher der pH-Wert im Bereich von 2 bis 6 liegt.

**30.** Verwendung einer Zusammensetzung, wie sie in einem der vorhergehenden Ansprüche definiert ist, bei der Herstellung einer kosmetischen Formulierung, die zum Schutz von Keratinfasern vor der Einwirkung von Stoffen der Atmosphäre bestimmt ist.

**31.** Verwendung einer Zusammensetzung, wie sie in einem der vorhergehenden Ansprüche definiert ist, als Formulierung zur Nachbehandlung von gefärbten Keratinfasern, insbesondere von durch Direktfärbung oder oxidative Färbung gefärbten Haaren.

**32.** Verfahren zum Färben von Keratinfasern und insbesondere der Haare, das darin besteht, auf die Fasern aufzubringen:

eine direktfärbende oder auf Oxidationsfärbung beruhende Färbezusammensetzung (A) während einer zur Entwicklung der Farbe ausreichenden Zeit und anschließend an diese Anwendung
eine Zusammensetzung (B), die aus einer Zusammensetz ung nach einem der vorhergehenden Ansprüche besteht,

wobei die Zusammensetzung (B) entweder unmittelbar anschließend oder zeitversetzt aufgebracht wird und die Anwendungen der Zusammensetzung zwischen zwei Färbungen wiederholt werden können.

**33.** Mehrkomponenten-Färbemittel, das mindestens eine erste Komponente, die eine direktfärbende oder auf Oxidationsfärbung beruhenden Färbezusammensetzung (A) und eine zweite Komponente aufweist, die eine Zusammensetzung (B) zur Nachbehandlung umfasst, die aus einer Zusammensetzung nach einem der vorhergehenden Ansprüche besteht.

**34.** Mehrkomponenten-Färbemittel, das enthält: mindestens eine erste Komponente, die eine Zusammen setzung ($A_1$) aufweist, die mindestens einen Direktfarbstoff enthält, eine zweite Komponente, die eine Zusammensetzung ($A_2$) umfasst, die mindestens ein Oxidationsmittel enthält, und eine dritte Komponente, die einen Zusammensetzung (B) zur Nachbehandlung umfasst, die aus einer Zusammensetzung nach einem der vorhergehenden Ansprüche besteht.

**35.** Mehrkomponenten-Färbemittel, das aufweist: mindestens eine erste Komponente, die eine Zusammensetzung ($A_1$) umfasst, die mindestens einen Vorläufer für ein Oxidationsfärbemittel enthält, eine zweite Komponente, die eine Zusammensetzung ($A_2$) umfasst, die mindestens ein Oxidationsmittel enthält und eine dritte Komponente, die eine Zusammensetzung (B) zur Vorbehandlung umfasst, die aus einer Zusammensetzung nach einem der vorhergehenden Ansprüche besteht.

## Claims

**1.** Cosmetic composition comprising, in a physiologically acceptable aqueous medium:

a) at least one agent for protecting keratin fibres, chosen from organic UV-screening agents in an amount ranging from 0.2% to 20% by weight relative to the total weight of the composition;

b) at least one aromatic alcohol which is a compound that is liquid at room temperature and atmospheric pressure, comprising at least one benzene or naphthalene ring and at least one alcohol function (OH) on at least one substituent of said ring, in an amount of greater than 1% by weight relative to the total weight of the composition;

c) at least one aromatic carboxylic acid chosen from:

benzoic acid
salicylic acid

and also the salified forms thereof, and mixtures thereof.

**2.** Composition according to Claim 1, in which the aqueous medium consists of at least water and contains at least one organic solvent other than aromatic alcohols and aromatic carboxylic acids.

**3.** Composition according to Claim 2, in which the said additional organic solvent is chosen from $C_1$-$C_4$ lower alcohols and polyols.

**4.** Composition according to Claim 2 or 3, in which the said organic solvent is ethanol.

**5.** Composition according to any one of Claims 1 to 4, in which the organic UV-screening agents are chosen from water-soluble or liposoluble, silicone or non-silicone screening agents.

**6.** Composition according to Claim 5, in which the organic UV-screening agents are chosen from dibenzoylmethane derivatives; anthranilates; cinnamic derivatives; salicylic derivatives; camphor derivatives; benzophenone derivatives; β, β-diphenyl-acrylate derivatives; triazine derivatives; benzotriazole derivatives; benzalmalonate derivatives; benzimidazole derivatives; imidazolines; bis-benzazolyl derivatives; p-aminobenzoic acid (PABA) derivatives; benzoxazoline derivatives; screening polymers and screening silicones; α-alkylstyrene-based dimers; 4,4-diarylbutadienes, and mixtures thereof.

**7.** Composition according to Claim 5 or 6, in which the liposoluble organic UV-screening agents are chosen from:

butylmethoxydibenzoylmethane,
ethylhexyl methoxycinnamate,
Homosalate,
ethylhexyl salicylate,
Octocrylene,
Benzophenone-3,
n-hexyl 2-(4-diethylamino-2-hydroxybenzoyl)benzoate,
4-methylbenzylidenecamphor,
ethylhexyl triazone,
bis-ethylhexyloxyphenol methoxyphenyl triazine,
diethylhexyl butamido triazone,
drometrizole trisiloxane,
Polysilicone-15,
1,1-dicarboxy(2,2'-dimethylpropyl)-4,4-diphenylbuta-diene,
2,4-bis[5-1(dimethylpropyl)benzoxazol-2-yl(4-phenyl)-imino]-6-(2-ethylhexyl)imino-1,3,5-triazine,

or mixtures thereof.

**8.** Composition according to Claim 6 or 7, in which the water-soluble organic UV-screening agents are chosen from:

PABA,
PEG-25 PABA
benzylidenecamphorsulfonic acid,
camphorbenzalkonium methosulfate,
terephthalylidenedicamphorsulfonic acid,
phenylbenzimidazolesulfonic acid,
disodium phenyldibenzimidazoletetrasulfonate,

Benzophenone-4,
Benzophenone-5.

**9.** Composition according to any one of Claims 1 to 8, in which the protecting agent (s) is (are) soluble in the aqueous medium of the composition.

**10.** Composition according to Claim 9, in which the protecting agent(s) has (have) a log P (octanol/water partition coefficient) of less than 9 and more preferentially less than 4.

**11.** Composition according to Claim 9 or 10, in which the protecting agent(s) is (are) soluble to at least 0.5% in water or $C_1$-$C_4$ lower alcohols such as ethanol, at 25°C.

**12.** Composition according to any one of Claims 1 to 11, in which the protecting agent (s) is (are) water-soluble organic UV-screening agents.

**13.** Composition according to Claim 12, in which the water-soluble UV-screening agent is Benzophenone-4.

**14.** Composition according to any one of Claims 1 to 13, in which the aromatic alcohol is chosen from:

benzyl alcohol
benzoylisopropanol
benzyl glycol
phenoxyethanol
dichlorobenzyl alcohol
methylphenylbutanol
phenoxyisopropanol
phenylisohexanol
phenylpropanol
phenylethyl alcohol
and mixtures thereof.

**15.** Composition according to Claim 14, in which the aromatic alcohol is benzyl alcohol.

**16.** Composition according to Claim 15, in which the aromatic carboxylic acid is benzoic acid.

**17.** Composition according to any one of Claims 1 to 16, in which the aromatic carboxylic acid(s) or salts thereof represent(s) from 0.001% to 30% by weight, preferably from 0.01% to 20% by weight and more particularly from 0.1% to 10% by weight relative to the total weight of the composition.

**18.** Composition according to any one of Claims 1 to 17, also containing one or more conditioning agents.

**19.** Composition according to Claim 18, in which the conditioning agent(s) represent(s) from 0.001% to 20% by weight, preferably from 0.01% to 10% by weight and more particularly from 0.1% to 3% by weight relative to the total weight of the composition.

**20.** Composition according to any one of Claims 1 to 19, also containing one or more anionic, cationic, amphoteric or nonionic surfactants.

**21.** Composition according to Claim 20, in which the surfactant(s) is (are) present in an amount of between 0.1% and 60%, preferably between 1% and 40% and even more preferentially between 5% and 30% by weight approximately relative to the total weight of the composition.

**22.** Composition according to any one of Claims 1 to 21, **characterized in that** it is in the form of a shampoo and comprises a washing base.

**23.** Composition according to Claim 22, in which the surfactant(s) forming the washing base represent(s) from 4% to 50% by weight, preferably from 6% to 35% by weight and even more preferentially from 8% to 25% by weight relative to the total weight of the composition.

**24.** Composition according to Claim 23, containing at least 3% by weight and more particularly from 4% to 30% by weight of anionic surfactant relative to the total weight of the composition.

**25.** Composition according to any one of the preceding claims, **characterized in that** it is in the form of an aqueous or aqueous-alcoholic lotion or in the form of a gel, a milk, a cream, an emulsion or a mousse.

**26.** Composition according to any one of the preceding claims, **characterized in that** it is packaged in the form of a vaporizer or a pump-dispenser bottle or in an aerosol container.

**27.** Composition according to any one of the preceding claims, **characterized in that** it is in the form of a rinse-out or leave-in hair conditioner or in the form of a composition to be applied after dyeing, bleaching, permanent-waving or relaxing the hair or alternatively between the two steps of a permanent-waving or hair-relaxing operation.

**28.** Composition according to any one of Claims 1 to 27, **characterized in that** the pH of the composition applied to the keratin fibres ranges from 1 to 11.

**29.** Composition according to Claim 28, in which the pH ranges from 2 to 6.

**30.** Use of a composition as defined in any one of the preceding claims in the preparation of a cosmetic formulation for protecting keratin fibres against the action of atmospheric agents.

**31.** Use of a composition as defined in any one of the preceding claims as a formulation for the post-treatment of dyed keratin fibres, in particular hair dyed by direct dyeing or by oxidation dyeing.

**32.** Process for dyeing keratin fibres and more particularly the hair, which consists in applying to the said fibres a direct or oxidation dye composition (A) for a time that is sufficient to develop the colour, and in following this application, after optionally rinsing and optionally drying, by the application of a composition (B) constituted of a composition according to any one of the preceding claims; the said composition (B) being applied either immediately or in a delayed manner, and the applications of the said composition possibly being repeated between two dyeing operations.

**33.** Multi-component colouring agent comprising at least one first component comprising a direct or oxidation dye composition (A) and a second component comprising a post-treatment composition (B) constituted of a composition according to any one of the preceding claims.

**34.** Multi-component colouring agent comprising at least one first component comprising a composition (A1) comprising at least one direct dye, a second component comprising a composition (A2) containing at least one oxidizing agent, and a third component comprising a post-treatment composition (B) constituted of a composition according to any one of the preceding claims.

**35.** Multi-component colouring agent comprising at least one first component comprising a composition (A1) containing at least one oxidation dye precursor, a second component comprising a composition (A2) containing at least one oxidizing agent, and a third component comprising a post-treatment composition (B) constituted of a composition according to any one of the preceding claims.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2627085 A
- EP 329032 A
- FR 2673839 A
- JP 5043437 A
- EP 669323 A
- US 2463264 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- EP 0337354 A
- FR 2270846 A
- FR 2383660
- FR 2598611
- FR 2470596
- FR 2519863
- FR 2505348
- FR 2542997
- EP 080976 A
- FR 2077143
- FR 2393573
- FR 2162025
- FR 2280361
- FR 2252840
- FR 2368508
- JP 1583363 A
- FR 2080759
- FR 2190406
- JP 2320330 A
- FR 2270846
- FR 2316271
- FR 2336434
- FR 2413907
- US 2273780 A
- US 2375853 A
- US 2388614 A
- US 2454547 A
- FR 3206462
- FR 2261002
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- FR 8516334 A
- US 4957732 A
- EP 186507 A
- EP 342834 A
- EP 412704 A
- EP 412707 A
- EP 640105 A
- WO 9500578 A
- EP 582152 A
- WO 9323009 A
- US 4693935 A
- US 4728571 A
- US 4972037 A
- DE 4424530
- DE 4424533
- DE 4402929
- DE 4420736
- WO 9523807 A
- WO 9407844 A
- EP 0646572 A
- WO 951666 A
- FR 2673179
- EP 0227994 A
- WO 9424097 A
- WO 9410131 A
- US 4874554 A
- US 4137180 A
- EP 486135 A
- WO 9311103 A
- US 2528378 A
- US 2781354 A
- FR 2692572
- WO 9501772 A
- WO 9515144 A
- EP 714954 A
- GB 1026978 A
- GB 1153196 A
- DE 2359399
- JP 63169571 A

- JP 05163124 B
- EP 0770375 A
- WO 9615765 A
- FR 2750048 A
- DE 3843892
- DE 4133957
- WO 9408969 A
- WO 9408970 A
- FR 2733749 A
- DE 19543988


**Littérature non-brevet citée dans la description**


- **WALTER NOLL.** Chemistry and Technology of Silicones. Academie Press, 1968
- **TODD ; BYERS.** Volatile Silicone fluids for cosmetics. *Cosmetics and toiletries,* vol. 91, 27-32
- **M.R. PORTER.** Handbook of Surfactants. Blackie & Son, 1991, 116-178